# EUROPEAN PATENT APPLICATION

(11) **EP 1 130 088 A1**
(43) Date of publication of application: **05.09.2001**
(21) Application number: 00105829.6
(22) Date of filing: 20.03.2000
(51) Int. Cl.: C12N 5/16, C12N 5/22, A61K 39/00

(54) **Hybrid cell vaccines**

(30) Priority: 27.02.2000 DE 10009030; 28.02.2000 US 185334 P
(71) Applicant: Eberhard-Karls-Universität Tübingen Universitätsklinikum, 72076 Tübingen (DE)
(72) Inventor: Kanz, Lothar, Prof.Dr., 72076 Tübingen (DE); Walden, Peter Ronald, Prof.Dr., 10405 Berlin (DE); Stuhler, Gernot, Dr., 72070 Tübingen (DE)
(74) Representative: Otten, Hajo, Dr.-Ing.

(57) **Abstract**

The present invention relates to methods and compositions for treating and preventing cancer and infectious disease using hybrid cells formed by fusion of allogeneic dendritic cells and autologous non-dendritic cells.

## Description

### 1. INTRODUCTION

The present invention relates to methods and compositions for treating and preventing cancer and infectious disease using hybrid cells formed by fusion of allogeneic dendritic cells and autologous non-dendritic cells.

### 2. BACKGROUND OF THE INVENTION

There is great interest in the development of an effective immunotherapeutic vaccine for treating or preventing human cancer. Success at such an immunotherapeutic approach will require the development of a vaccine that is both extremely specific for the target tumor or infected cell, and, at the same time, capable of eliciting a very strong immune response.

### 2.1 ANTIGEN PRESENTATION AND THE IMMUNE RESPONSE

Cells of the immune system arise from pluripotent stem cells through two main lines of differentiation, the lymphoid lineage and the myeloid lineage. The lymphoid lineage produces lymphocytes, such as T cells, B cells, and natural killer cells, while the myeloid lineage produces monocytes, macrophages, and neutrophils and other accessory cells, such as dendritic cells, platelets, and mast cells. Cytotoxic T lymphocytes and helper T cells develop and undergo selection in the thymus, distinguished by the presence of one of two surface markers, CD4 (helper T cells) or CD8 (CTLs).

Antigens are processed by two distinct routes depending upon whether their origin is intracellular or extracellular. Intracellular or endogenous protein antigens are presented to CD8⁺ CTLs by class I major histocompatibility complex (MHC) molecules, expressed in most cell types, including tumor cells. CD8⁺ CTLs are particularly important in resisting cancer and pathogens, as well as rejecting allografts (Terstappen et al., 1992, Blood 79:666-677). On the other hand, extracellular antigenic determinants are presented on the cell surface of "specialized" or "professional" APCs, such as dendritic cells and macrophages, for example, by class II MHC molecules to CD4⁺ "helper" T cells (see generally, W.E. Paul, ed., Fundamental Immunology. New York: Raven Press, 1984).

Class I and class II MHC molecules are the most polymorphic proteins known. A further degree of heterogeneity of MHC molecules is generated by the combination of class I and class II MHC molecules, known as the MHC haplotype. In humans, HLA-A, HLA-B and HLA-C, three distinct genetic loci located on a single chromosome, encode class I molecules. Because T cell receptors specifically bind complexes comprising antigenic peptides and the polymorphic portion of MHC molecules, T cells respond poorly when an MHC molecule of a different genetic type is encountered. This specificity results in the phenomenon of MHC-restricted T cell recognition and T cell cytotoxicity.

Antigens are taken up in the periphery by the antigen-presenting cells (APCs) and migrate to secondary organs, such as the spleen and the lymph nodes, where they interact with circulating lymphocytes. Interaction between T lymphocytes and APCs triggers several effector pathways, including activation of CD8⁺ cytotoxic T lymphocytes (CD8⁺ CTLs) and resulting in antigen-specific interactions such as T cell production of cytokines including interleukin-1 (IL-1), interferon-γ, interleukin-2 (IL-2), interleukin-4 (IL-4), interleukin-7 (IL-7), and interleukin-12 (IL-12), produced by CD4⁺ helper T cells or dendritic cells. Additional antigen-specific interactions include the recognition of plasma-membrane-bound costimulatory molecule, such as B7, which is present on the antigen-presenting cell membrane by a co-receptor on the cell surface of helper T cells, called CD28, a member of the Ig superfamily. In addition to antigen-specific interactions during antigen presentation, antigen non-specific adhesive mechanisms also operate. These stabilize the binding of T lymphocytes to APC. Receptor molecules on APC, such as ICAM-1/CD54, LFA-3/CD58, and B7, bind corresponding co-receptors on T cells. Thus, helper T cells receiving such antigen-specific signals are activated to proliferate and to secrete a variety of interleukins. CTLs receiving such signals are activated to kill target cells that carry the same class I MHC molecule and the same antigen that originally induced their activation. However, T cells receiving cytokine signals in the absence of costimulation become anergized, leading to tolerance (Lamb et al., 1983, J. Exp. Med. 157:1434-1447; Mueller et al., 1989, Annu. Rev. Immunol. 7:445-480; Schwartz, 1992, Cell 71:1065-1068; Mueller and Jenkins, 1995, Curr. Opin. Immunol. 7:375-381).

### 2.2 IMMUNOTHERAPY AGAINST CANCER

The cytotoxic T cell response is the most important host response for the control of growth of antigenic tumor cells (Anichimi et al., 1987, Immunol. Today 8:385-389). Studies with experimental animal tumors as well as spontaneous human tumors have demonstrated that many tumors express antigens that can induce an immune response. Some antigens are unique to the tumor, and some are found on both tumor and normal cells. Several factors influence the immunogenicity of the tumor, including, for example, the specific type of carcinogen involved, and immunocompetence of the host and the latency period (Old et al., 1962, Ann. N.Y. Acad. Sci. 101:80-106; Bartlett, 1972, J. Natl. Cancer. Inst. 49:493-504). It has been demonstrated that T cell-mediated immunity is of critical importance for rejection of virally and chemically induced tumors (Klein et al., 1960, Cancer Res. 20:1561-1572; Tevethia et al., 1974, J. Immunol. 13:1417-1423).

Adoptive immunotherapy for tumors refers to the therapeutic approach wherein immune cells with antitumor reactivity are administered to a tumor-bearing host, with the objective that the cells cause the regression of an established tumor, either directly or indirectly. Immunization of hosts bearing established tumors, as well a spontaneous tumors, with tumor cells or tumor antigenshas often been ineffective since the tumor may have already elicited an immunosuppressive response (Greenberg, 1987, Chapter 14, in Basic and Clinical Immunology, 6th ed., ed. by Stites, Stobo and Wells, Appleton and Lange, pp. 186-196; van der Bruggen et al., 1991, Science 254:1643-7). Thus, prior to immunotherapy, it had been necessary to reduce the tumor mass and deplete all the T cells in the tumor-bearing host (Greenberg et al., 1983, page 301-335, in "Basic and Clinical Tumor Immunology", ed. Herbermann RR, Martinus Nijhoff).

Animal models have been developed in which hosts bearing advanced tumors can be treated by the transfer of tumor-specific syngeneic T cells (Mulé et al., 1984, Science 225:1487-1489). Investigators at the National Cancer Institute (NCI) have used autologous reinfusion of peripheral blood lymphocytes or tumor-infiltrating lymphocytes (TIL), T cell cultures from biopsies of subcutaneous lymph nodules, to treat several human cancers (Rosenberg, S.A., U.S. Patent No. 4,690,914, issued September 1, 1987; Rosenberg et al., 1988, N. Engl. J. Med., 319:1676-1680). For example, TIL expanded *in vitro* in the presence of IL-2 have been adoptively transferred to cancer patients, resulting in tumor regression in select patients with metastatic melanoma. Melanoma TIL grown in IL-2 have been identified as CD3⁺ activated T lymphocytes, which are predominantly CD8+ cells with unique *in vitro* anti-tumor properties. Many long-term melanoma TIL cultures lyse autologous tumors in a specific class I MHC- and T cell antigen receptor-dependent manner (Topalian et al., 1989, J. Immunol. 142:3714).

Application of these methods for treatment of human cancers would entail isolating a specific set of tumor-reactive lymphocytes present in a patient, expanding these cells to large numbers *in vitro,* and then putting these cells back into the host by multiple infusions. Since T cells expanded in the presence of IL-2 are dependent upon IL-2 for survival, infusion of IL-2 after cell transfer prolongs the survival and augments the therapeutic efficacy of cultured T cells (Rosenberg et al., 1987, N. Engl. J. Med. 316:889-897). However, the toxicity of the high-dose IL-2 and activated lymphocyte treatment has been considerable, including high fevers, hypotension, damage to the endothelial wall due to capillary leak syndrome, and various adverse cardiac events such as arrhythmias and myocardial infarction (Rosenberg et al., 1988, N. Engl. J. Med. 319:1676-1680). Furthermore, the demanding technical expertise required to generate TILs, the quantity of material needed, and the severe adverse side effects limit the use of these techniques to specialized treatment centers.

CTLs specific for class I MHC-peptide complexes could be used in treatment of cancer and viral infections, and ways have been sought to generate them *in vitro* without the requirement for priming *in vivo*. These include the use of dendritic cells pulsed with appropriate antigens (Inaba et al., 1987, J. Exp. Med. 166:182-194; Macatonia et al., 1989, J. Exp. Med. 169:1255-1264; De Bruijn et al., 1992, Eur. J. Immunol. 22:3013-3020). RMA-S cells (mutant cells expressing high numbers of 'empty' cell surface class I MHC molecules) loaded with peptide (De Bruijn et al., 1991, Eur. J. Immunol. 21:2963-2970; De Bruijn et al., 1992, *supra;* Houbiers et al., 1993, Eur. J. Immunol. 26:2072-2077) and macrophage phagocytosed-peptide loaded beads (De Bruijn et al., 1995, Eur. J. Immunol. 25, 1274-1285).

Fusion of B cells or dendritic cells with tumor cells has been previously demonstrated to elicit anti-tumor immune responses in animal models (Guo et al., 1994, Science, 263:518-520; Stuhler and Walden, 1994, Cancer Immunol. Immuntother. 1994, 39:342-345; Gong et al., 1997, Nat. Med. 3:558-561; Celluzzi, 1998, J. Immunol. 160:3081-3085; Gong, PCT publication WO 98/46785, dated October 23, 1998). In particular, immunization with hybrids of tumor cells and antigen presenting cells has been shown to result in protective immunity and rejection of established tumors in various rodent models.

Thus, despite such recent efforts, as yet, no effective immunotherapeutic vaccine against human cancer and infectious disease has been described for humans.

Citation or discussion of a reference herein shall not be construed as an admission that such is prior art to the present invention.

### 3. SUMMARY OF THE INVENTION

The present invention relates to methods for treating and preventing cancer and infectious disease using hybrid cells formed by fusion of allogeneic dendritic cells and autologous non-dendritic cells. The invention is based, in part, on the discovery and demonstration that hybrid cells of allogeneic mature dendritic cells (DCs) and autologous tumor cells are potent and highly effective vaccines against human cancer. Such hybrid cells combine the vigorous alloreactivity of mature DCs with the specific antigenicity of autologous tumor cells, thereby eliciting a highly specific and vigorous CTL response.

In one embodiment, the invention provides a method of treating or preventing a condition in a mammal selected from the group consisting of cancer and an infectious disease which comprises administering to a mammal in need of said treatment or prevention a therapeutically effective amount of a hybrid cell formed by the fusion of an allogeneic dendritic cell and a non-dendritic cell, which has the same class I MHC haplotype as said mammal.

In an alternative embodiment, the invention provides a method of treating a condition in a mammal selected from the group consisting of cancer and an infectious disease which comprises administering to a mammal in need of such treatment a therapeutically effective amount of a hybrid cell formed by the fusion of an autologous dendritic cell and an allogeneic non-dendritic cell, in such an embodiment the non-dendritic cell may be obtained from a cell line. In a preferred embodiment, the dendritic cell is a mature dendritic cell.

In one embodiment, the dendritic cell is a mature dendritic cell.

In another embodiment, the non-dendritic cell is a tumor cell obtained from the mammal. In another embodiment, the non-dendritic cell is a tumor cell line derived from a tumor cell obtained from the mammal in which the hybrid cell is to be administered.

In another embodiment, the non-dendritic cell is a recombinant cell transformed with one or more antigens that display the antigenicity of a tumor-specific antigen.

In another embodiment, the non-dendritic cell is a recombinant cell transformed with one or more antigens that display the antigenicity of an antigen of an infectious agent.

In another embodiment, the mammal is a human.

In another embodiment, the cancer is selected from the group consisting of renal cell carcinoma, fibrosarcoma, myxosarcoma, liposarcoma, chondrosarcoma, osteogenic sarcoma, chordoma, angiosarcoma, endotheliosarcoma, lymphangiosarcoma, lymphangioendotheliosarcoma, synovioma, mesothelioma, Ewing's tumor, leiomyosarcoma, rhabdomyosarcoma, colon carcinoma, pancreatic cancer, breast cancer, ovarian cancer, prostate cancer, squamous cell carcinoma, basal cell carcinoma, adenocarcinoma, sweat gland carcinoma, sebaceous gland carcinoma, papillary carcinoma, papillary adenocarcinomas, cystadenocarcinoma, medullary carcinoma, bronchogenic carcinoma, hepatoma, bile duct carcinoma, choriocarcinoma, seminoma, embryonal carcinoma, Wilms' tumor, cervical cancer, testicular tumor, lung carcinoma, small cell lung carcinoma, bladder carcinoma, epithelial carcinoma, glioma, astrocytoma, medulloblastoma, craniopharyngioma, ependymoma, pinealoma, hemangioblastoma, acoustic neuroma, oligodendroglioma, meningioma, melanoma, neuroblastoma, retinoblastoma, leukemias, acute lymphocytic leukemia, acute myelocytic leukemia; chronic leukemia, polycythemia vera, lymphoma, multiple myeloma, Waldenström's macroglobulinemia, and heavy chain disease.

In another embodiment, the invention provides a method for preventing a cancer in an individual having a predisposition for the cancer wherein the non-dendritic cell displays the antigenicity of an antigen associated with the cancer. In one embodiment, the cancer is colon cancer, and the non-dendritic cell is derived from a cell or cell line displaying an antigen associated with colon cancer. In another embodiment, the cancer is breast cancer, and the non-dendritic cell is derived from a cell or a cell line displaying an antigen associated with breast cancer.

In another embodiment, the invention provides a method for making a fusion with a mature dendritic cell and a non-dendritic comprising: (a) subjecting a population of allogeneic dendritic cells and a population of autologous non-dendritic cells obtained from a mammal to conditions that promote cell fusion, and (b) inactivating the population of hybrid cells. In another embodiment, the cell fusion is accomplished by electrofusion. In another embodiment, inactivating the population of hybrid cells is accomplished by γ-irradiating the cells. In a preferred embodiment, the invention provides a method for making a fusion of a mature human dendritic cell and a non-dendritic cell allogeneic to the dendritic cell, with the proviso that said non-dendritic cell is freshly isolated or obtained from a primary cell culture, and is not obtained from an established cell line.

In another embodiment, the invention provides for hybrid cells comprising a mature dendritic cell that is fused to a non-dendritic cell, which is freshly isolated or from a primary cell culture, wherein the dendritic cell and the non-dendritic cell are allogeneic to each other. In a preferred embodiment, both the dendritic and non-dendritic cells are human. The present invention also encompasses a population of such hybrid cells, wherein at least 10% - 15% of the cells are fused, and preferably 15% - 20% of the cells are fused.

In another embodiment, the invention provides a kit comprising, in one or more containers, a sample containing a population of mature dendritic cells and instructions for its use in treating or preventing cancer or an infectious disease. In another embodiment, the kit further comprising a cuvette suitable for electrofusion. In another embodiment, the dendritic cells are cryopreserved.

### 4. BRIEF DESCRIPTION OF THE FIGURES

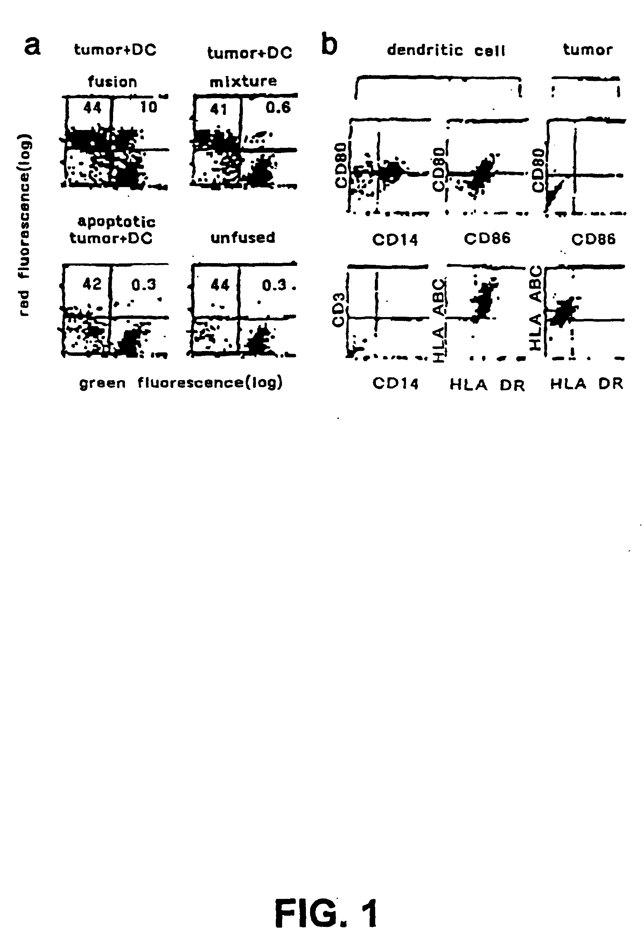

**FIG. 1A-B** Characterization of one hybrid cell vaccine. **A.** Generation of hybrids. DCs and tumor cells were labeled with red and green fluorescent dyes. Tumor cells fused with DCs (tumor-DC fusion) or a mixture of DC-DC fusions and tumor-tumor fusions were analyzed by flow cytometry. Uptake of apoptotic bodies was assessed 24 hr. after incubation of apoptotic tumor cells with DCs. Unfused cells (lower right), control. Numbers (upper quadrants). Percentage of cells per quadrant. **B.** Phenotype of DCs and tumor cells before electrofusion determined by flow cytometry analysis using monoclonal antibodies labeled with fluorescein isothiocyanate and phycoerythrin. Monoclonal antibodies against CD83/CD1a, CD80/CD86, CD3/CD14, and HLA ABC/HLA DR were used for DC analysis; monoclonal antibodies against CD80/CD86 and HLA ABC/ HLA DR were used for tumor cell analysis.

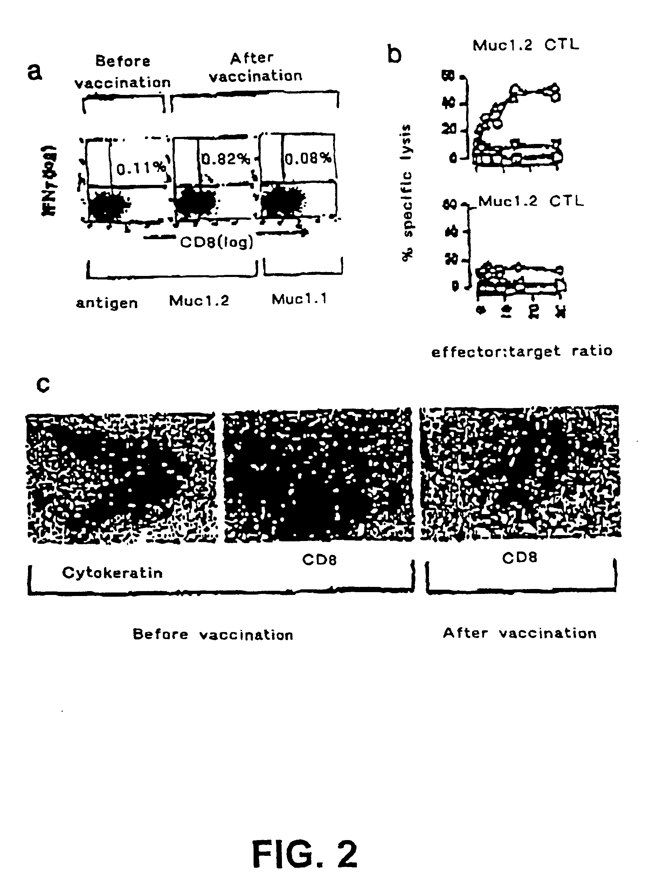

**FIG. 2A-B** Immunological response data. **A.** Specificity of T lymphocytes from patient 7 for Muc1. and Muc1.2 tumor antigens, analyzed before and after vaccination. Lymphocytes were stimulated *ex vivo* with the peptide antigens for 6h and production of IFNγ was visualized by intracellular staining with specific phycoerythrin-labeled antibodies. Correlation with lymphocyte subpopulations was accomplished by 3-color flow cytometry analysis using antibodies against CD3 and conjugated to peridinine chlorophyll protein, and against CDE, conjugated to fluorescein isothiocyanate. Gates were set on CD- T lymphocytes. **B.** HLA-A2-positive mononuclear cells from patient 7 were stimulated *in vitro* for seven days with Mucl1.2 (top) or Muc1.1 (bottom) epitopes and were subsequently re-stimulated for additional 5 days in the presence of the antigens and 20 U/ml interleukin-2. CTL activity was determined by specific lysis of ⁵¹CR-labeled target cells (10⁴ cells) at varying effector to target ratios. Targets included: HLA-A2-positive but Muc1-negative Croft target cells coated with Muc1.1 (Croft Muc1.1; ◇), Muc1.2 (Croft Muc1.2; Δ) or the irrelevant HIV (Croft HIV; □) peptide antigen; A498 (circles), HLA-A2-and Muc1-positive renal cell carcinoma tumor cell line; SKOV (∇), an HLA-A2-negative but Muc1-positive cell line; and K362 (◁), used to analyze natural killer cell activity. **C.** Immunohistochemical analysis of the DTH challenge site. Tumor cells and CD8⁺ T lymphocytes were visualized in serial subcutaneous sections using monoclonal antibodies against cytokeratin and CD8 antigens. Before (left and middle), tumor cell detritus and moderate infiltration of CD8⁻ lymphocytes in biopsies before vaccination therapy, after (right), infiltration of CD8⁺ CTL into the DTH challenge sight after vaccination, original magnification, x 400.

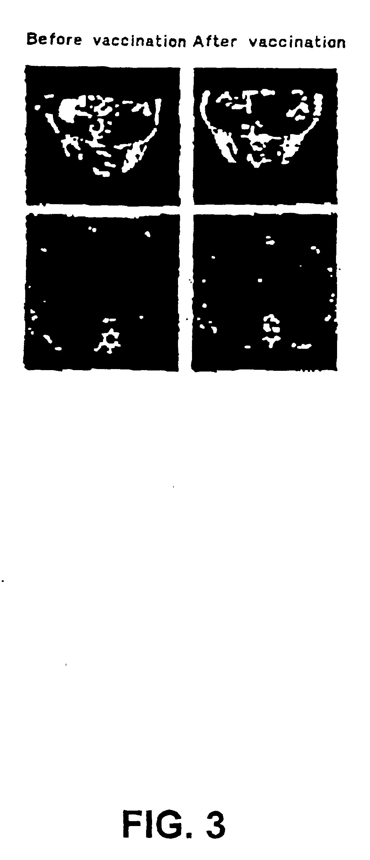

**FIG. 3** Clinical response to hybrid cell vaccination. CT scans of patient 8 before (left) and 6 months after (right) hybrid cell vaccination show rejection of bone (top) and lung (bottom) metastasis. Arrows, sites of lesions before vaccination.

### 5. DETAILED DESCRIPTION OF THE INVENTION

The invention provides methods and compositions for therapeutic vaccines against cancer and infectious disease, produced by fusion of allogeneic dendritic cells with autologous non-dendritic cells. Allogeneic dendritic cells, particularly terminally differentiated dendritic cells, are highly immunogenic. Using the methods described herein, allogeneic dendritic cells can be fused to a non-dendritic cell containing an antigen of interest, such as a cancer antigen. The resulting hybrids of dendritic cells and non-dendritic cells can be used as a potent vaccine against a condition involving such an antigen, such as a cancer. This approach is particularly advantageous when a specific antigen is not readily identifiable, as in the case of many cancers. For treatment of human cancer, for example, non-dendritic cells can be obtained directly from the tumor of a patient. Hybrid cell vaccines prepared in this way are highly specific for the individual tumor being treated.

Described below, are compositions and methods relating to such immunotherapeutic vaccines. In particular, Sections 5.1, 5.2, and 5.3 describe the non-dendritic, dendritic, and the hybrid cells, respectively, that are used with the invention, and methods for their isolation, preparation, and/or generation. Target cancers and infectious diseases that can be treated or prevented using such vaccines are described below in Sections 5.4 and 5.5. Section 5.6 describes the methods and use of these hybrid cells as therapeutic vaccines against cancer and infectious disease.

### 5.1 NON-DENDRITIC CELLS

A non-dendritic cell of the present invention can be any cell bearing an antigen of interest for use in a hybrid cell vaccine. Such non-dendritic cells may be isolated from a variety of desired subjects, such as a tumor of a cancer patient or a subject infected with an infectious disease. The methods for isolation and preparation of the non-dendritic cells are described in detail hereinbelow.

The source of the non-dendritic cells may be selected, depending on the nature of the disease with which the antigen is associated. Preferably, the non-dendritic cells are autologous to the subject being treated, *i.e.,* the cells are obtained from cells of the ultimate target cells *in vivo* (*e.g*., of the tumor cells of the intended recipient that it is desired to inhibit). In this way, since whole cancer cells or other non-dendritic cells may be used in the present methods, it is not necessary to isolate or characterize or even know the identities of these antigens prior to performing the present methods. However, any non-dendritic cell can be used as long as at least one antigen present on the cell is an antigen specific to the target cells, and as long as the non-dendritic cell has the same class I MHC haplotype as the mammal being treated.

For treatment or prevention of cancer, the non-dendritic cell is a cancer cell. In this embodiment, the invention provides hybrid cells that express antigens expressed by cancer cells, *e.g*., tumor-specific antigens and tumor associated antigens, and are capable of eliciting an immune response against such cancer cells. In one embodiment of the invention, any tissues, or cells isolated from a cancer, including cancer that has metastasized to multiple sites, can be used for the preparation of non-dendritic cells. For example, leukemic cells circulating in blood, lymph or other body fluids can also be used, solid tumor tissue (*e.g*., primary tissue from a biopsy) can be used. Examples of cancers that are amenable to the methods of the invention are listed in Section 5.4, infra.

In a preferred embodiment, the non-dendritic cells of the invention may be isolated from a tumor that is surgically removed from a human patient who will be the recipient of the hybrid cell vaccines. Prior to use, solid cancer tissue or aggregated cancer cells should be dispersed, preferably mechanically, into a single cell suspension by standard techniques. Enzymes, such as but not limited to, collagenase and DNase may also be used to disperse cancer cells. In yet another preferred embodiment, the non-dendritic cells of the invention are obtained from primary cell cultures, i.e., cultures of original cells obtained from the body. Typically, approximately 1x10⁶ to 1x10⁹ non-dendritic cells are used for formation of hybrid cells.

In one embodiment, approximately 1 x 10⁶ to 1 x 10⁹ non-dendritic cells are used for formation of hybrid cells. In another embodiment, 5 x 10⁷ to 2 x 10⁸ cells are used. In a specific embodiment, approximately 5 x 10⁷ non-dendritic cells are used to form hybrid cells.

Cell lines derived from cancer cells or infected cells or tissues can also be used as non-dendritic cells, provided that the cells of the cell line have the same antigenic determinant(s) as the antigen of interest on the cancer or infected cell or tissue to be treated. Cancer or infected tissues, cells, or cell lines of human origin are preferred.

In an alternative embodiment, in order to prepare suitable non-dendritic cells that are cancer cells, noncancerous cells, preferably of the same cell type as the cancer desired to be treated or prevented can be isolated from the recipient or, less preferably, other individual who shares at least one MHC allele with the intended recipient, can be treated such that the treated noncancer cell expresses a tumor-specific antigen having the antigenicity of an antigen present on the tumor or cancer to be treated or prevented. In one aspect of this embodiment, noncancerous cells can be treated with agents that cause the particular or a similar cancer or a transformed state; such agents may include but not limited to, radiation, chemical carcinogens, and viruses. Standard techniques can be used to treat the cells and propagate the cancer or transformed cells so produced.

Alternatively, if the gene encoding a tumor-specific antigen, tumor-associated antigen or antigen of the pathogen is available, normal cells of the appropriate cell type from the intended recipient or an individual having at least one common MHC allele may be transformed or transfected *in vitro* with an expression construct containing the gene such that the antigen is expressed in the recipient's cells. Optionally, more than one such antigen may be expressed in the recipient's cell in this fashion, as will be appreciated by those skilled in the art, any techniques known, such as those described in Ausubel et al. (eds., 1989, Current Protocols in Molecular Biology, Greene Publishing Associates and Wiley Interscience, New York), may be used to perform the transformation or transfection and subsequent recombinant expression of the antigen gene in recipient's cells. These non-dendritic cells bearing one or more MHC molecules in common with the recipient are suitable for use in the methods for formation of hybrid cells of the invention.

In another embodiment, the methods of the invention can be used to prevent a cancer in an individual having a predisposition for a particular type of cancer, such as, for example, an individual member of a high risk group or family having a genetic predisposition for the disease. For example, for the prevention of cancer, non-autologous cells that share at least one MHC allele with the individual to be treated, may be obtained from a tumor or cancer from a second individual, a cell line, or from recombinant cells transfected with a tumor-specific antigen. In one aspect of this embodiment, for example, such non-autologous cells may be obtained from an established cell line expressing a common antigen to the type of cancer to be prevented. Such a cell line, for example, may be derived from a cancer cell of a family member having previously had the cancer. In one embodiment, a hybrid cell vaccine made by this method is used to prevent or protect against familial breast cancer. In another embodiment, a hybrid cell vaccine made by this method is used to prevent or protect against colon cancer.

In another embodiment, for the treatment and prevention of infectious disease, an antigen having the antigenicity of a pathogen, in particular, an intracellular pathogen, such as a virus, bacterium, parasite, or protozoan, can be used. In one embodiment, for example, a cell that is infected with a pathogen is used. In another embodiment, a cell that recombinantly expresses an antigen having the antigenicity of the pathogen is used. An exemplary list of infectious diseases that can be treated or prevented by the methods of the invention is provided in Section 5.5, below.

The non-dendritic cells used for the generation of hybrid cells and the target tumor or pathogen infected cell must have at least one common MHC allele in order to elicit an immune response in the mammal. The more MHC alleles in common, the more preferred the method. Most preferred is where the non-dendritic cells are derived from the intended recipient (*i.e.,* are autologous). Less preferred, the non-dendritic cells are nonautologous, but share at least one MHC allele with the cancer cells of the recipient. If the non-dendritic cells are obtained from the same or syngeneic individual, such cells will all have the same class I MHC haplotype. If they are not all obtained from the same subject, the MHC haplotype can be determined by standard HLA typing techniques well known in the art, such as serological tests and DNA analysis of the MHC loci. An MHC haplotype determination does not need to be undertaken prior to carrying out the procedure for generation of the hybrid cells of the invention.

Non-dendritic cells, such as cells containing an antigen having the antigenicity of a cancer cell or an infectious disease cell, can be identified and isolated by any method known in the art. For example, cancer or infected cells can be identified by morphology, enzyme assays, proliferation assays, or the presence of cancer-causing viruses. If the characteristics of the antigen of interest are known, non-dendritic cells can also be identified or isolated by any biochemical or immunological methods known in the art. For example, cancer cells or infected cells can be isolated by surgery, endoscopy, other biopsy techniques, affinity chromatography, and fluorescence activated cell sorting (*e.g.*, with fluorescently tagged antibody against an antigen expressed by the cells).

There is no requirement that a clonal or homogeneous or purified population of non-dendritic cells be used. A mixture of cells can be used provided that a substantial number of cells in the mixture contain the antigen or antigens present on the tumor cells being targeted. In a specific embodiment, the non-dendritic cells and/or dendritic cells are purified.

### 5.2 DENDRITIC CELLS

Dendritic cells can be isolated or generated from blood or bone marrow, or secondary lymphoid organs of the subject, such as but not limited to spleen, lymph nodes, tonsils, Peyer's patch of the intestine, and bone marrow, by any of the methods known in the art. Preferably, DCs used in the methods of the invention are mature (or terminally differentiated) dendritic cells. The source of dendritic cells is preferably human blood monocytes.

Immune cells obtained from such sources typically comprise predominantly recirculating lymphocytes and macrophages at various stages of differentiation and maturation. Dendritic cell preparations can be enriched by standard techniques (see e.g., Current Protocols in Immunology, 7.32.1-7.32.16, John Wiley and Sons, Inc., 1997). In one embodiment, for example, DCs may be enriched by depletion of T cells and adherent cells, followed by density gradient centrifugation. DCs may optionally be further purified by sorting of fluorescence-labeled cells, or by using anti-CD83 MAb magnetic beads.

Alternatively, a high yield of a relatively homogenous population of DCs can be obtained by treating DC progenitors present in blood samples or bone marrow with cytokines, such as granulocyte-macrophage colony stimulating factor (GM-CSF) and interleukin 4 (IL-4). Under such conditions, monocytes differentiate into dendritic cells without cell proliferation. Further treatment with agents such as TNFα stimulates terminal differentiation of DCs.

By way of example but not limitation, mature dendritic cells can be obtained from blood monocytes as follows: peripheral blood monocytes are obtained by standard methods (see, e.g., Sallusto et al., 1994, J. Exp. Med. 179:1109-1118). Leukocytes from healthy blood donors are collected by leukapheresis pack or buffy coat preparation using Ficoll-Paque density gradient centrifugation and plastic adherence. Cells are allowed to adhere to plastic dishes for 4 hours at 37°C. Nonadhering cells are removed and adherent monocytes are cultured for 7 days in culture media containing 0.1µg/ml granulocyte-monocyte colony stimulating factor and 0.05µg/ml interleukin-4. In order to prepare mature dendritic cells, tumor necrosis factor-α (TNF-α) is added, preferably on day 5, and cells are collected on day 7.

Dendritic cells obtained in this way characteristically express the cell surface marker CD83. In addition, such cells characteristically express high levels of MHC class II molecules, as well as cell surface markers CD1α, CD40, CD86, CD54, and CD80, but lose expression of CD14. Other cell surface markers characteristically include the T cell markers CD2 and CD5, the B cell marker CD7 and the myeloid cell markers CD13, CD32 (FcγR II), CD33, CD36, and CD63, as well as a large number of leukocyte-associated antigens

Optionally, standard techniques such as morphological observation and immunochemical staining, can be used to verify the presence of dendritic cells. For example, the purity of dendritic cells can be assessed by flow cytometry using fluorochrome-labeled antibodies directed against one or more of the characteristic cell surface markers noted above, *e.g*., CD83, HLA-ABC, HLA-DR, CD1α, CD40, and/or CD54. This technique can also be used to distinguish between mature and immature DCs, using fluorochrome-labeled antibodies directed against CD14, which is present in immature, but not mature DCs.

### 5.3 GENERATION OF HYBRID CELLS

Next, non-dendritic cells are fused to allogeneic DCs. Cells can optionally be sterilely washed prior to fusion. Fusion can be accomplished by any cell fusion technique, many of which are known in the art, provided that the fusion technique used results in a mixture of fused cells suitable for injection into a mammal for treatment or prevention of cancer or infectious disease. Preferably, electrofusion is used. Electrofusion techniques are well known in the art (Stuhler and Walden, 1994, Cancer Immunol. Immunother. 39: 342-345; see Chang et al. (eds.), Guide to Electroporation and Electrofusion. Academic Press, San Diego, 1992).

In a preferred embodiment, the following protocol is used. In the first step, approximately 5 x 10⁷ tumor cells and 5 x 10⁷ dendritic cells (DCs) are suspended in 0.3 M glucose and transferred into an electrofusion cuvette. The sample is dielectrophoretically aligned to form cell-cell conjugates by pulsing the cell sample at 100 V/cm for 5-10 secs. Optionally, alignment may be optimized by applying a drop of dielectrical wax onto one aspect of the electroporation cuvette to 'inhomogenize' the electric field, thus directing the cells to the area of the highest field strength. In a second step, a fusion pulse is applied. Various parameters may be used for the electrofusion. For example, in one embodiment, the fusion pulse may be from a single to a triple pulse. In another embodiment, electrofusion is accomplished using from 500 to 1500V/cm, preferably, 1200V/cm at about 25 µF.

The extent of hybrid cell formation within a population of antigenic and dendritic cells can be determined by a number of diagnostic techniques known in the art. In one embodiment, for example, hybrids are characterized by emission of both colors after labeling of DCs and tumor cells with red and green intracellular fluorescent dyes, respectively. Samples of DCs without tumor cells, and tumor cells without DCs can be used as negative controls, as well as tumor + DC mixture without electrofusion.

Before introduction of the hybrid cell vaccine into a patient, the hybrid cells are inactivated so as to prevent the tumor cells from proliferating, for example, by irradiation. In a preferred embodiment, cells are irradiated at 200 Gy, and injected, preferably without further selection. In one embodiment, the hybrid cells prepared by this method comprise approximately 10 and 20% of the total cell population. In yet another embodiment, the hybrid cells prepared by this method comprise approximately 5 to 50% of the total cell population. In an alternative embodiment, the cell population can be enriched for hybrid cells, by any cell selection or sorting technique known in the art.

### 5.4 TARGET CANCERS

The cancers and oncogenic diseases that can be treated or prevented using the hybrid cells of the invention of the present invention include, but are not limited to: human sarcomas and carcinomas, *e.g.,* renal cell carcinoma, fibrosarcoma, myxosarcoma, liposarcoma, chondrosarcoma, osteogenic sarcoma, chordoma, angiosarcoma, endotheliosarcoma, lymphangiosarcoma, lymphangioendotheliosarcoma, synovioma, mesothelioma, Ewing's tumor, leiomyosarcoma, rhabdomyosarcoma, colon carcinoma, pancreatic cancer, breast cancer, ovarian cancer, prostate cancer, squamous cell carcinoma, basal cell carcinoma, adenocarcinoma, sweat gland carcinoma, sebaceous gland carcinoma, papillary carcinoma, papillary adenocarcinomas, cystadenocarcinoma, medullary carcinoma, bronchogenic carcinoma, hepatoma, bile duct carcinoma, choriocarcinoma, seminoma, embryonal carcinoma, Wilms' tumor, cervical cancer, testicular tumor, lung carcinoma, small cell lung carcinoma, bladder carcinoma, epithelial carcinoma, glioma, astrocytoma, medulloblastoma, craniopharyngioma, ependymoma, pinealoma, hemangioblastoma, acoustic neuroma, oligodendroglioma, meningioma, melanoma, neuroblastoma, retinoblastoma; leukemias, *e.g.*, acute lymphocytic leukemia and acute myelocytic leukemia (myeloblastic, promyelocytic, myelomonocytic, monocytic and erythroleukemia); chronic leukemia (chronic myelocytic (granulocytic) leukemia and chronic lymphocytic leukemia); and polycythemia vera, lymphoma (Hodgkin's disease and non-Hodgkin's disease), multiple myeloma, Waldenström's macroglobulinemia, and heavy chain disease.

### 5.5 TARGET INFECTIOUS DISEASES

The infectious diseases that can be treated or prevented using the hybrid cells of the invention of the present invention include those caused by intracellular pathogens such as viruses, bacteria, protozoans, and intracellular parasites. Viruses include, but are not limited to viral diseases such as those caused by hepatitis type B virus, parvoviruses, such as adeno-associated virus and cytomegalovirus, papovaviruses such as papilloma virus, polyoma viruses, and SV40, adenoviruses, herpes viruses such as herpes simplex type I (HSV-I), herpes simplex type II (HSV-II), and Epstein-Barr virus, poxviruses, such as variola (smallpox) and vaccinia virus, RNA viruses, including but not limited to human immunodeficiency virus type I (HIV-I), human immunodeficiency virus type II (HIV-II), human T-cell lymphotropic virus type I (HTLV-I), and human T-cell lymphotropic virus type II (HTLV-II); influenza virus, measles virus, rabies virus, Sendai virus, picornaviruses such as poliomyelitis virus, coxsackieviruses, rhinoviruses, reoviruses, togaviruses such as rubella virus (German measles) and Semliki forest virus, arboviruses, and hepatitis type A virus.

In another embodiment, bacterial infections can be treated or prevented such as, but not limited to disorders caused by pathogenic bacteria including, but not limited to, *Streptococcus pyogenes, Streptococcus pneumoniae, Neisseria gonorrhoea, Neisseria meningitidis, Corynebacterium diphtheriae, Clostridium botulinum, Clostridium perfringens, Clostridium tetani, Haemophilus influenzae, Klebsiella pneumoniae, Klebsiella ozaenae, Klebsiella rhinoscleromotis, Staphylococcus aureus, Vibrio cholerae, Escherichia coli, Pseudomonas aeruginosa, Campylobacter* (Vibrio) *fetus, Campylobacter jejuni, Aeromonas hydrophila, Bacillus cereus, Edwardsiella tarda, Yersinia enterocolitica, Yersinia pestis, Yersinia pseudotuberculosis, Shigella dysenteriae, Shigella flexneri, Shigella sonnei, Salmonella typhiimurium, Salmonella typhii, Treponema pallidum, Treponema pertenue, Treponema carateneum, Borrelia vincentii, Borrelia burgdorferi, Leptospira icterohemorrhagiae, Mycobacterium tuberculosis, Toxoplasma gondii, Pneumocystis carinii, Francisella tularensis, Brucella abortus, Brucella suis, Brucella melitensis, Mycoplasma spp., Rickettsia prowazeki, Rickettsia tsutsugumushi, Chlamydia spp., and Helicobacter pylori.*

In another preferred embodiment, the methods can be used to treat or prevent infections caused by pathogenic protozoans such as, but not limited to, *Entomoeba histolytica, Trichomonas tenas, Trichomonas hominis, Trichomonas vaginalis, Trypanosoma gambiense, Trypanosoma rhodesiense, Trypanosoma cruzi, Leishmania donovani, Leishmania tropica, Leishmania braziliensis, Pneumocystis pneumonia, Plasmodium vivax, Plasmodium falciparum, and Plasmodium malaria.*

### 5.6 PHARMACEUTICAL PREPARATIONS AND METHODS OF ADMINISTRATION

### 5.6.1 VACCINE FORMULATIONS

The vaccine formulations of the invention comprise an effective immunizing amount of the hybrid cells. Suitable preparations of hybrid cell vaccines include injectables, preferably as a liquid solution.

Many methods may be used to introduce the vaccine formulations of the invention; these include but are not limited to subcutaneous injection, intralymphatically, intradermal, intramuscular, intravenous, and via scarification (scratching through the top layers of skin, *e.g.*, using a bifurcated needle).

Preferably, hybrid cell vaccines are injected intradermally (intracutaneously) or intralymphatically at a site distant from the site of the tumor or the infection. Injection in these locations elicit the most potent immune response, since dendritic cells are activated on the surface, for example, the skin, and migrate into the lymph nodes. Furthermore, injection near the tumor or infected area itself is not recommended, since this area is surrounded by a tumor-protective array of cytokines. In one preferred embodiment, for example, hybrid cells are injected intralymphatically, *i.e.* in the lymph nodes or the lymphatic draining system. In one embodiment, for example, hybrid cells are injected in the lymph vessels on the dorsal side of the foot.

In addition, if desired, the vaccine preparation may also include minor amounts of auxiliary substances such as wetting or emulsifying agents, pH buffering agents, and/or compounds which enhance the effectiveness of the vaccine. The effectiveness of an auxiliary substances may be determined by measuring the induction of antibodies directed against a hybrid cell.

The mammal to which the vaccine is administered is preferably a human, but can also be a non-human animal including but not limited to cows, horses, sheep, pigs, fowl (*e.g.*, chickens), goats, cats, dogs, hamsters, mice and rats.

### 5.6.2 EFFECTIVE DOSE

The vaccines can be administered to a patient at therapeutically effective doses to treat or prevent cancer or infectious disease. A therapeutically effective amount refers to that amount of the hybrid cells sufficient to ameliorate the symptoms of such a disease or disorder, such as, e.g., regression of a tumor. Effective doses (immunizing amounts) of the vaccines of the invention may also be extrapolated from dose-response curves derived from animal model test systems. The precise dose of hybrid cells to be employed in the vaccine formulation will also depend on the particular type of disorder being treated. For example, if a tumor is being treated, the aggressiveness of the tumor is an important consideration when considering dosage. Other important considerations are the route of administration, and the nature of the patient. Thus the precise dosage should be decided according to the judgment of the practitioner and each patient's circumstances, e.g., depending upon the immune status of the individual patient, according to standard clinical techniques.

In a preferred embodiment, for example, to treat a human tumor, a hybrid cell vaccine formed by cells of the tumor fused to allogeneic DCs at a site away from the tumor, and preferably near the lymph tissue. The vaccine may be repeated after an appropriate interval, *e.g*., every 2 to 12 weeks, preferably, every 3 to 8 weeks, using between approximately 10⁶ -10⁹, preferably about 5 x 10⁷ - 1 x 10⁸, hybrid cells per administration.

The present invention thus provides a method of immunizing a mammal, or treating or preventing cancer or infectious disease in a mammal, comprising administering to the mammal a therapeutically effective amount of a hybrid cell vaccine of the present invention.

### 5.6.3 KITS

The invention further provides kits for facilitating delivery of the immunotherapeutic according to the methods of the invention. The kits described herein may be conveniently used, *e.g.*, in clinical settings to treat patients exhibiting symptoms of cancer of an infectious disease. In one embodiment, for example, a kit is provided comprising, in one or more containers: a) a sample of a population of human dendritic cells and b) instructions for its use in a method for treating or protecting against cancer or an infectious disease. An ampule of sterile diluent can be provided so that the ingredients may be mixed prior to administration. In another embodiment the kit further comprises a cuvette suitable for electrofusion. In one embodiment, the dendritic cells are cryopreserved.

### 5.6.4 DETERMINATION OF IMMUNOGENICITY OF HYBRID CELL VACCINES

Optionally, the hybrid cell vaccines can be assayed for immunogenicity using any method known in the art. By way of example but not limitation, one of the following three procedures can be used.

### 5.6.4.1 MLTC ASSAY

Optionally, the hybrid cell vaccines may be tested for immunogenicity using a MLTC assay. For example, 1x10⁷ hybrid cells are γ-irradiated, and mixed with T lymphocytes. At various intervals the T lymphocytes are tested for cytotoxicity in a 4 hour ⁵¹Cr-release assay (see Palladino et al., 1987, Cancer Res. 47:5074-5079). In this assay, the mixed lymphocyte culture is added to a target cell suspension to give different effector:target (E:T) ratios (usually 1:1 to 40:1). The target cells are pre-labeled by incubating 1x10⁶ target cells in culture medium containing 500 µCi of ⁵¹Cr per ml for one hour at 37°C. The cells are washed three times following labeling. Each assay point (E:T ratio) is performed in triplicate and the appropriate controls incorporated to measure spontaneous ⁵¹Cr release (no lymphocytes added to assay) and 100% release (cells lysed with detergent). After incubating the cell mixtures for 4 hours, the cells are pelleted by centrifugation at 200g for 5 minutes. The amount of ⁵¹Cr released into the supernatant is measured by a gamma counter. The percent cytotoxicity is measured as cpm in the test sample minus spontaneously released cpm divided by the total detergent released cpm minus spontaneously released cpm.

In order to block the MHC class I cascade a concentrated hybridoma supernatant derived from K-44 hybridoma cells (an anti-MHC class I hybridoma) is added to the test samples to a final concentration of 12.5%.

### 5.6.4.2 ANTIBODY RESPONSE ASSAY

In one embodiment of the invention, the immunogenicity of hybrid cells is determined by measuring antibodies produced in response to the vaccination, by an antibody response assay, such as an enzyme-linked immunosorbent assay (ELISA) assay. Methods for such assays are well known in the art (see, *e.g*., Section 2.1 of Current Protocols in Immunology, Coligan et al. (eds.), John Wiley and Sons, Inc. 1997). In one mode of the embodiment, microtitre plates (96-well Immuno Plate II, Nunc) are coated with 50 µl/well of a 0.75 µg/ml solution of a purified cancer cell or infected used in the vaccine in PBS at 4°C for 16 hours and at 20°C for 1 hour. The wells are emptied and blocked with 200 µl PBS-T-BSA (PBS containing 0.05% (v/v) TWEEN 20 and 1% (w/v) bovine serum albumin) per well at 20°C for 1 hour, then washed 3 times with PBS-T. Fifty µl/well of plasma or CSF from a vaccinated animal (such as a model mouse or a human patient) is applied at 20°C for 1 hour, and the plates are washed 3 times with PBS-T. The antigen antibody activity is then measured calorimetrically after incubating at 20°C for 1 hour with 50µl/well of sheep anti-mouse or anti-human immunoglobulin, as appropriate, conjugated with horseradish peroxidase diluted 1:1,500 in PBS-T-BSA and (after 3 further PBS-T washes as above) with 50 µl of an o-phenylene diamine (OPD)-H₂O₂ substrate solution. The reaction is stopped with 150 µl of 2M H₂SO₄ after 5 minutes and absorbance is determined in a photometer at 492 nm (ref. 620 nm), using standard techniques.

### 5.6.4.3 CYTOKINE DETECTION ASSAYS

The CD4+ T cell proliferative response to the hybrid cell vaccine may be measured by detection and quantitation of the levels of specific cytokines. In one embodiment, for example, intracellular cytokines may be measured using an IFN-γ detection assay to test for immunogenicity of the hybrid cell vaccine. In an example of this method, peripheral blood mononuclear cells from a patient treated with the hybrid cell vaccine are stimulated with peptide antigens such as mucin peptide antigens or Her2/neu derived epitopes. Cells are then stained with T cell-specific labeled antibodies detectable by flow cytometry, for example FITC-conjugated anti-CD8 and PerCP-labeled anti-CD4 antibodies. After washing, cells are fixed, permeabilized, and reacted with dye-labeled antibodies reactive with human IFN-γ (PE- anti-IFN-γ). Samples are analyzed by flow cytometry using standard techniques.

Alternatively, a filter immunoassay, the enzyme-linked immunospot assay (ELISPOT) assay, may be used to detect specifc cytokines surrounding a T cell. In one embodiment, for example, a nitrocellulose-backed microtiter plate is coated with a purified cytokine-specific primary antibody, *i.e.,* anti-IFN-γ, and the plate is blocked to avoid background due to nonspecific binding of other proteins. A sample of mononuclear blood cells, containing cytokine-secreting cells, obtained from a patient vaccinated with a hybrid cell vaccine, is diluted onto the wells of the microtitre plate. A labeled, *e.g.,* biotin-labeled, secondary anti-cytokine antibody is added. The antibody cytokine complex can then be detected, *i.e.* by enzyme-conjugated streptavidin - cytokine-secreting cells will appear as "spots" by visual, microscopic, or electronic detection methods.

### 5.6.4.4 TETRAMER STAINING ASSAY

In another embodiment, the "tetramer staining" assay (Altman et al., 1996, Science 274: 94-96) may be used to identify antigen-specific T-cells. For example, in one embodiment, an MHC molecule containing a specific peptide antigen, such as a tumor-specific antigen, is multimerized to make soluble peptide tetramers and labeled, for example, by complexing to streptavidin. The MHC-peptide antigen complex is then mixed with a population of T cells obtained from a patient treated with a hybrid cell vaccine. Biotin is then used to stain T cells which express the antigen of interest, *i.e.,* the tumor-specific antigen.

### 6. EXAMPLE: Vaccination with Tumor Cell-Dendritic Cell Hybrids Results in Regression of Human Metastatic Renal Cell Carcinoma

This Example demonstrates the successful use of hybrid cells as vaccines for treating cancer in a study of 17 patients with renal cell carcinoma. The hybrid cells used in this study were formed by electrofusion of autologous cells derived from renal cell carcinoma tissue with allogeneic dendritic cells. After 13 months, four patients completely rejected all metastatic tumor lesions, one presented a mixed response, and two had a tumor mass reduction of greater than 50%. This Example also demonstrates induction of HLA-A2-restricted cytotoxic T cells reactive with the Muc1 tumor-associated antigen and recruitment of CD8+ lymphocytes into tumor challenge sites. The results indicate that hybrid cell vaccination is a safe and effective therapy for renal cell carcinoma and provides a broadly applicable strategy for other malignancies with unknown antigens.

### 6.1 MATERIALS AND METHODS

Patients. The study was conducted at the University of Gottingen and approved by the Clinical Institutional Ethical Review Board of University of Gottingen. Patients with metastatic renal cell carcinoma staged PT1-4NxM1 were enrolled in this study after informed consent was obtained. Inclusion criteria were bi-dimensionally measurable metastatic lesions, and ECOG score of less than 3, life expectancy of more than 3 months and a positive delayed-type hypersensitivity test for common recall antigens including intact cellular immune reactivity (Multitest Merieux, Leimen, Germany). Patients with brain metastasis or second malignancy or participants in other clinical trials were excluded from this study. Patient recruitment started in January 1998, and the study ended in November/December 1999. Seventeen patients were enrolled (Table 1 and 2).

Treatment. All patients had tumor nephrectomy or metastatic surgery at the University of Göttingen, Urology Department or at an affiliated hospital. Medical histories and physical examinations were obtained before each vaccination. Eligible patients received at least two subcutaneous injections with hybrids in close proximity to inguinal lymph nodes with a 6-week interval. Patients with measurable clinical responses (described below) after 12 weeks received a booster vaccination every three months.

Clinical response and toxicity data. Metastatic lesions were evaluated by computerized tomography (CT) scans, ultrasonic examinations and bone scontigraphy before and twelve weeks after the first immunization. Patients not progressing clinically received CT scans every three months (Table 1 and 2, latest CT scan data). World Health Organization definitions were used for complete response (no metastatic lesion detectable) and partial response (greater than 50% reduction of all tumor sights) for at least 3 months. Stable disease was defined as a change of less than 25 percent in size and numbers for 6 weeks. Adverse effects were assessed according to World Health Organization criteria. Blood and urine were routinely tested. CD4:CD8 ratios were determined and lymphocyte subsets were analyzed using CD3-, CD4-, CD8-, CD19 and CD56- reactive monoclonal antibodies and flow cytometry (Becton, Dickinson and Co., Franklin Lakes, New Jersey).

Tumor and dendritic cells. Tumor samples were processed within 12 hours after tumor nephrectomy or surgery of metastasis. Single-cell suspensions were obtained by mincing the tumor and then digesting the sample with 5.6 mg collagenase type VIII and 0.26 mg DNAse type IV in 10 ml RPMI medium per grams of tumor (Sigma). To confirm the epithelial origin of tumor cells, co-expression of vimentin and cytokeratin 8 and 18 was evaluated by immunocytochemistry according to manufacturers' instructions (Dako, Ontario, Canada). Before electrofusion the MHC class 1 expression and ploidy of the tumor cells were determined by flow cytometry. DCs were generated from peripheral blood monocytes as described (Sallusto et al., 1994, J. Exp. Med. 179:1109-1118). Leukocytes from healthy blood donors were collected by leukaphoresis. After Ficoll-Hypoque density centrifugation, cells were allowed to adhere to plastic dishes for 4 hours at 37°C. Nonadhering cells were removed and adherent monocytes were cultured for 7 days in X-Vivo 15 medium (BioWhitthaker, Walkersville, Maryland) containing 0.1 µg/ml granulocyte-monocyte colony stimulating factor and 0.05 µg/ml interleukin-4. Tumor necrosis factor-α was added during the last 2 days of the cultures at a concentration of 0.5 µg/ml (all cytokines from Genzyme, Cambridge, Massachusetts). The purity of DCs thus obtained was greater 85%, as assessed by flow cytometry (FacsCalibur, Becton, Dickinson and Co., Franklin Lakes, New Jersey) using fluorochrome-labeled antibodies directed against HLA-ABC, HLA-DR, CD1α, CD3, CD14, CD19, CD40, CD54 AND CD83 (all from Becton, Dickinson and Co., Franklin Lakes, New Jersey, except for antibodies against CD83 and HLA-ABC, Immunotech, Westbook, Maine).

Generation of the hybrid cell vaccine. Autologous tumor cells and allogeneic DCs (5x10⁷ each) were washed, resuspended in 0.3 M glucose and transferred into an electoelution cuvette (BioRad, Richmond, California). Electrofusion was accomplished by first aligning the cells to form cell-cell conjugates at 100 V/cm for 5-10 secs. Alignment was optimized by applying a drop of dielectrical wax onto one aspect of the electroporation cuvette to 'inhomogenize' the electric field, thus directing the cells to the area of the highest field strength, in a second step, a pulse of 1,200V/cm at 25 µF was applied to fuse the aligned cells using a Gene Pulser II (BioRad, Richmond, California). The hybrids were irradiated (200 Gy), and injected without further selection. To determine fusion efficacy, CDs and tumor cells were strained with CellTracker red and green fluorescent dyes (Molecular Probes, Eugene, Oregon) and analyzed by flow cytometry (Becton, Dickinson and Co., Franklin Lakes, New Jersey). Apoptosis of tumor cells was induced by UV irradiation using a Stratalinker device (Stratagene, La Jolla, California).

Intracellular detection of IFN-γ. Peripheral blood mononuclear cells (PBMCs; 2x10⁵) were stimulated for 6 hours at 37°C in 200 µl RPMI medium with 50 µg/ml mutin-derived peptide antigens Mucl.1 (STPPVHNV ) or Mucl.2 (LLLLTVLTV); (Brossart, P. et al., 1999, Blood 93:4309-4317), or the Her-2/neu-derived epitopes GP2 (IISAVVGIL or E75 (KIGSFLAFL); (Brossart et al., 1998, Cancer Res. 58:732-736). Brefeldin A (10 mM; Sigma) was added during the last 3 hours, cells were stained with fluorescein isothiocyanate- conjugated monoclonal antibody against CD8 and peridinine chlorophyll protein-labeled monoclonal antibody against CD4 (Becton, Dickinson and Co., Franklin Lakes, New Jersey), After being washed, cells were fixed with 2% formaldehyde/phosphate-buffered saline for 15 mins at room temperature. Cells were permeabilized in a 0.5% saponin, 2% bovine serum albumin in phosphate-buffered saline, and phycoerythrin-labeled antibodies against human IFN-γ (PharMingen, San Diego, California) were added for 30 min. at room temperature. The samples were analyzed using a FacsCalibur flow cytometry device (Becton, Dickinson and Co., Franklin Lakes, New Jersey).

Cytotoxicity assay. PBMCs (0.5 x 10⁶) were cultured in RPMI medium containing 10% fetal calf serum and antibiotics with 1 x10⁴ irradiated autologous PBMCs pulsed with 50 µg/ml Muc1.1 or Muc1.2. After 7 days, cells were re-stimulated for additional 5 days. In the presence of 2.5 x 10⁶ antigen-pulsed autologous PBMCs and 20 U/ml interleukin-2 (Genzyme, Cambridge, Massachusetts). HLA-A2-restricted mucin-specific lysis was measured in a standard ⁵¹Cr-release assay. ⁵¹Cr-labeled target cells were added to a varying number of effector cells for 6 hours, and supernatants were assayed for ⁵¹C. Targets used were the HLA-A2 and Muc1-positive renal cell carcinoma cell line A-498, the HLA-A2-negative but Muc1-positive ovarian cell line HLA-negative but natural-killer cell-sensitive pro-erythroblastic cell line K562. The MLA-A2 positive but Muc1-negative B-cell line Croft was coated with 50 µg/ml Muc1.1, Muc1.2 or the irrelevant CTL epitope HIV-RT (ILKEPVHGV) before being labeled with ⁵¹C. The percentage of specific lysis alter 6 hours of incubation of effector and target cells was calculated as 100 x (experimental release-spontaneous release)/ (maximum release-spontaneous release).

**Table 1**

| **Patients Characteristics Before and After Hybrid Cell Vaccination Therapy** | | | | | | | |
|---|---|---|---|---|---|---|---|
| | | Patient | | Donor | Tumor before vaccination | | |
| Pat. No. | Age | HLA patient | Previous Therapy | HLA donor | stage/grade | Site of mets | Muc1 |
| 1 | 66 | A1,11.88 DR17.4 | TN | A24 2B,B7.57 A2,25.B44.39 | T3bNxM1C2 | Ly, B | +++ |
| 2 | 63 | A1,3,857.35 DR4,11 | TN, CH | A2,11.B62.49 A1,3B7.57 | T3NxM1G2 | L, B | +++ |
| 3 | 80 | A1,88 DR15.7 | TN,S | A11,2B,B44.39 A2,25,B44.39 | T4N2M1G3 | L, LR | + |
| 4 | 68 | A2,31,860 DR4 | TN | A1,2,B13.57 A2,3,B7.47 | T3bNxM1G2 | L | +++ |
| 5 | 67 | A2,835 DR15.11 | TN.S | A1,2.B13.57 A2,3,B7.47 | T3NxM1G3 | Ly ,U | Neg |
| 6 | 57 | A2.88 DR23 | TN.S | A2,11,B39.51 A2,24,B7.60 | T3NxM1G3 | L, B, U, LR | ++ |
| 7 | 60 | A2,26.87.57 DR4.13 | TN | A1,2,B13.57 A1,3.B7 57 | T3bNxMlG3 | L | +++ |
| 8 | 37 | A3,24.835 DR1.15 | TN | A11,2,B13.57 A1,11,B3.62 | T2NxM1G2 | L, B | + |
| 9 | 73 | A2.87 DR4.13 | TN | A1,2,B13.57 A1.A3.B7 BB | T3bNxM1G2 | L | Neg |
| 10 | 53 | A24.32.88.44 DR16.17 | TN.S | A1,11,BB,62 A2,31,B62,49 | T4NxM1G3 | L, U, L R | +++ |
| 11 | 63 | A1,3,857 DR15.17 | TN | A1,2,B13.57 A2,31,B62.49 | T4NxM1G3 | L | ++ |
| 12 | 61 | A23.87.57 DR2.15 | TN | A231,B62.49 A1,3B7.57 | T4NxM1G3 | L R, L, B | ++ |
| 13 | 70 | A1,24,862.35 DR13 | TN | A2,3B7.47 A26.31.B3B.60 | T4NxMlG3 | L,B,Li, Pa | ++ |
| 14 | 43 | A24.11.87.52 DRB.15 | TN | A1,3,B7.57 A24,2B,B7.57 | T3bNxMlG2 | L, S T, U | +++ |
| 15 | 67 | A3,25,813.27 DR1.15 | TN.S | A26,31,B3B.60 A24,2B,B7.57 | T3NxMlG2 | L | +++ |
| 16 | 67 | A1,88 DR15 | TN | A24,2B,B7.57 A2,24,B7.60 | T3bNxM1G2 | L, LR, 8 | +++ |
| 17 | 68 | A1,885 DR11.15 | TN.S | A3,B7,1B A1,2,B62.60 | T3NxM1G3 | LR, L, U | Neg |
| Ch, chemotherapy with IFN, interleukin-2 and 5-fluorouracil; S, surgery of metastasis, TN, tumor naphrectomy. Donor HLA class 1 haplotypes for the first two vaccinations. B, bone; L, lung; Li, liver; LR, local recurrence ; Ly, lymph nodes; Pa, Pancreas; ST, soft tissue, Muc1 expression of the primary tumor, as assessed by Immune Histochemistry. Percent positive cells: 0, neg.; 1-10% + 10-60%, ++; 60-100%, +++. DTH reactions after challenges with irradiated autologous tumor cells; + erythema and induration greater than 6mm in diameter. Clinical response: CR, complete response; PR, partial response; MR 'mixed response'; SD, stable disease; PD, progressive disease; DFD, died from disease. Follow-up. (FU), in months/date of latest CT scan in which the clinical response was documented. | | | | | | | |

Immunohistochemistry used standard procedures. Serial paraffin sections were stained with monoclonal antibodies against CD8 (C8/144B) or pancytokeratin (MNF116; both from Dako, Carpinteria, California). Muc1 was detected with the monoclonal antibody CA-15-3 (BioGenex, San Ramon, California). Primary antibodies were detected using the Envision-perozidase system (Dako, Carpinteria, California).

### 6.2 RESULTS

For rapid and large-scale generation of hybrids, electrofusion was established as a two-step procedure. As many as 10⁸ cells can be processed simultaneously using this technique without the need for tumor cell culture or fusion reagents. In the first step, tumor cells and dendritic cells (DCs) were dielectrophoretically aligned to form cell-cell conjugates. In the second step, a fusion pulse was applied, yielding 10-15% hybrid cell formation (FIG. 1A). Hybrids are characterized by emission of both colors after labeling of DC s and tumor cells with red and green intracellular fluorescent dyes, respectively. No double-flourescent cells were detected in experiments analyzing either a mixture of DCs fused to DCs or tumor cells fused to tumor cells (FIG. 1A, tumor + DC mixture), or without electrofusion (FIG. 1A, unfused). Uptake of apoptotic bodies from tumor cells by DCs is distinct from hybrid cell formation, as the incubation of DCs with apoptotic tumor cells for 24 hours did not result in double-fluorescent cells (FIG. 1A apoptotic tumor + DC).

Monocyte-derived DCs were used as fusion partners for the tumor cells because of their unique ability to induce naive T lymphocytes *in vitro* as well as *in vivo.* The phenotype of the DCs typically included substantial CD1a and CD83 expression and the absence of CD14 molecules (FIG. 1B). Although MHC class I (HLA ABC) antigens are expressed by tumor and dendritic cells, we detected high levels of the co-stimulatory molecules CD80 and CD86 and MHC class II (MLA-DR) antigens for recruitment of helper T cells only on DCs. The fusion of the two populations may thus merge the unique and tumor-specific antigens with the co-stimulatory capabilities of the DC (Gong et al., 1997, Nature Med. 3:558-561). Staining of tumor-DC fusions with fluorochrome-labeled antibodies directed against surface molecules or flourescent membrane dyes led to a high number of false-positive hybrid cells, because particles of ruptured membranes generated during the electrofusion process or apotosis adhere to unfused cells. As the requisite involvement of helper T cells for CTL induction is well established in hybrid cell vaccination studies (Stuhler, G. & Walden, 1994, P. Cancer Immunol. Immunother. 39: 342-345), allogeneic DCs were used to recruit allo-reactive helper T cells, thereby avoiding the need of additional HLA class II-restricted tumor antigens (Brossart, P. et al., Blood 93:4309-4317 1999).

Seventeen patients were vaccinated subcutaneously with fusions generated from 5 x 10⁷ autologous tumor cells and 5 x 10⁷ allogeneic DCs in close proximity to inguinal lymph nodes (patient characteristics, table 1 and 2). After electrofusion and irradiation with 200 Gy, the vaccine was immediately injected, without further selection. Identical, freshly-prepared booster vaccinations were administered after six weeks, followed by a re-evaluation of the patients six weeks later. All patients not suffering progressive disease received continuous booster immunizations after three months. Different HLA-mismatched donors were recruited for each immunization to enable cognate tumor-associated antigen recognition by avoiding dominant allospecific responses.

The hybrid cell vaccination was well tolerated by all patients. There were no serious adverse effects or any clinical signs of autoimmune reactions. In some patients, mild fever for one to two days or a transient erythema and induration at the sight of injection occurred. Six patients reported pain at metastatic sights that was treated sufficiently with non-opioid analgesics. There were no substantial changes in the results of routine blood tests or in the ratios of lymphocyte populations in peripheral blood after multiple immunizations, as determined by flow cytometry using reagents specific for CD3, CD4, CD8, CD19 and CD86. Thus, hybrid cell vaccination using electrofusion techniques can be applied repeatedly without substantial toxicity.

To analyze effector cells involved in tumor cell rejection and to define the specificity of the immune reaction induced we use the previously identified HLA-A2 restricted Mucl (Brossart et al., 1999, Blood 93:4309-4313) and HER2/neu (Brossart et al., 1998, Cancer Res. 58:732-736) epitopes. In keeping with published data (Fujita et al., 1999, Brit. J. Cancer 80: 301-308), Mucl and HER2/new expression by the primary tumor was found in 82% (Table 1) and 0% of the patients, respectively. There was substantial HER2/new expression in tumor cell lines established from patients 4, 5, 6, 7 and 16. There was specific gamma interferon (IFN-γ) production by CD8⁺ T lymphocytes of HLA-A2-positive patients before and after vaccination as shown by stimulation of peripheral T lymphocytes with respective tumor peptide antigens *ex vivo* and intracellular induction of cytokine, as detected by flow cytometry. No reactivity was found against Mucl. 1 and Muc1.2 (Brossart et al., 1999, supra) or the HER2/neu derived antigens GP2 and E75 (Brossart et al., 1998, supra) before hybrid cell vaccination therapy. Patient 7, however, had a tumor - specific reaction after the fourth booster vaccination as 0.82% of all CD8⁺ CTLs stained positive for IFN-γ after *in vitro* stimulation with Muc1.2 peptide antigens for six hours (FIG. 2A). No reactivity was found against Muc1.1, E75 or GP2 epitopes. There was a similar response, with 0.4% Muc1.2-reactive CTLs after hybrid cell vaccination in patient 4, whose disease has been stable for eighteen months after an initial regression of less than 50% of some but not all LUNG metastases. These results, thus, demonstrate induction of specific CTLs and the establishment of a tumor-directed immune response *in vivo*, in keeping with data showing reversal of tolerance and Muc1-specific tumors immunity in HLA-A2-transgenic mice (Brossart, P. et al., 1999, Blood 93:4309-4317). Regardless of clinical responses, however, Muc1 - or HER-2/new-specific CTLs were not present in other two HLA-A2-positive patients (9 and 12), indicating involvement of T lymphocytes with specificities for antigens other than the epitopes tested here.

Lymphocytes from patient 7 were next stimulated *in vitro* with the Muc1.1, Muc1.2 and the irrelevant HIV epitope, and then tested them for specific lytic activity in a ⁵¹Cr-release assay. The HLA-A2-positive but Muc1-negative cell line Croft cells were used, pulsed with the peptide antigens, as a target. CTLs stimulated in the presence of Muc1.2 specifically lysed Muc1.2-pulsed but not HIV-pulsed Croft cells (FIG. 2B). Although induction of low-affinity CTLs against Muc1 was expected, considerable lysis of the HLA-A2 and Muc1-positive renal cell carcinoma line A498 was found. HLA-A2 restriction of the CTL response was confirmed by the finding that the HLA-A2-negative but Muc1-positive cell line SKOV was not lysed. Muc1.2 stimulated CTLs did not lyse Muc1.1-coated Croft or A498 cells, indicating immune dominance of Muc1.2 over Muc1.1 in patient 7. There was no lysis in either assay of the K562 line, which is sensitive to natural killer cells. The possibility of *in vitro* induction of CTL reactivity was excluded by the finding that CTLs stimulated with HIV epitopes did not lyse HIV-coated CROFT target cells. Although CTLs specific for the self antigen Muc1.2 were present *in vitro,* patient 7 did not present any signs of autoimmune disease, and the function of these CTLs *in vivo* remains unclear. The observation that only 60-80% of tumor cells from patient 7 expressed mucin antigens indicates involvement of other antigens not tested here.

To monitor the 'homing' of lymphocytes into tumor sites and to further analyze lymphocyte populations involved in tumor cell destruction, delayed type hypersensitivity (DTH) tests were performed by injecting 5 x 10⁵ irradiated autologous tumor cells intracutaneously in the forearm. No positive DTH reactions were obtained before vaccination. However, 11 of 17 patients tested after 12 weeks presented a positive reaction greater than 6 mm in diameter 1-3 days after tumor challenge. Except for patients 4, 9, 10 and 17, all patients developing positive DTH reactions had a clinical response of complete or partial rejection of metastatic lesions. Patient 2, who did not develop a positive skin reaction after chemotherapy with IFN, interleukin-2 and 5-fluorouracil, partially responded to vaccination therapy after a delay.

Histological analyses of skin biopsies before hybrid vaccination therapy showed cytokeratin-positive tumor cells and a moderate lymphocytic infiltration corresponding to the primary tumor lesion (FIG. 2C). However, accumulation of CD8⁺ lymphocytes was found at the tumor challenge site only after immunizations.

At the conclusion of the study, seven of the seventeen patients (41%) responded to hybrid cell vaccination, with four complete tumor remissions, two partial remissions, and one 'mixed response'. In addition, the multiple pulmonal lesions of another two patients (4 and 9) were stabilized for 17 and 15 months, respectively. The mean follow-up time was 13 months (range, 3-21 months). Three of the four patients with complete remission successfully rejected all metastases within the first 12 weeks with a total of two injections and remained free of any detectable tumor lesions for up to 21 months. Typically, the reduction of the tumor mass occurred within the first weeks after the first immunization. Metastases of the lung, the bones, lymph nodes and soft tissues were successfully eradicated. In general, patients suffering large tumor burden did poorly (patients 6, 10, 13, and 17). However, the presence of large tumor masses did not necessarily limit vaccination success, as patients 12 and 16 rejected local recurrent tumor lesions larger than 500 ml. Despite regression of the local recurrence, however, there was progression of bone metastases in patient 12, which was classified as a 'mixed response'. There were initial partial responses in three patients with tumor mass reduction greater than 50% after 12 weeks: Patient 16 remained in partial remission; patient 8 completely rejected all metastatic tumor sites after 122 months (FIG. 3, regression in lung and bones); and patient 14 had an initial partial regression, but metastases of the liver eventually progressed and the patient died from the disease. All other patients (8 of 17) suffered progressive disease or died from their disease.

The data presented herein indicate that hybrid cell vaccination is a safe and effective immune therapy for human metastatic renal cell carcinoma. This vaccination is an example of an immune therapeutic strategy that is individualized regardless of the patient's genetic status, when shared or unique antigens are presented by the tumor. Given the heterogenicity of tumor cells within one lesion (Sallusto et al., 1994, J. Exp. Med. 179:1109-1118), a strategy aimed at indiction of CTLs directed against multiple and different tumor associated antigens may account for the results obtained. Moreover, using allogeneic dendritic cells as fusion partners for tumor cells improves vaccination success compared with a study using activated B cells (Guo, Y. et al., 1994, Science 263:518-520).

The invention is not to be limited in scope by the specific embodiments described which are intended as single illustrations of individual aspects of the invention, and functionally equivalent methods and components are within the scope of the invention. Indeed various modifications of the invention, in addition to those shown and described herein will become apparent to those skilled in the art from the foregoing description and accompanying drawings. Such modifications are intended to fall within the scope of the appended claims.

All references cited herein are incorporated by reference herein in their entireties for all purposes.

**Table 2**

| **Patients Characteristics Before and After Hybrid Cell Vaccination Therapy** | | | | | | | |
|---|---|---|---|---|---|---|---|
| | Vaccination | | | | Response to vaccination | | |
| Patient | First vaccinations | No. of vaccinations | Adverse effects | DTH switch | Staging after 12 weeks | Outcome | FU/CT- |
| 1 | Feb 98 | 7 | no | + | CR | CR | 19/Sep 99 |
| 2 | Feb 98 | 6 | no | - | SD | PR | 21/Nov 99 |
| 3 | Mar 98 | 3 | fever | - | SD | PD | 18/Jun 98 |
| 4 | May 98 | 6 | no | + | SD | SD | 17/Oct 99 |
| 5 | May 98 | 2 | no | - | SD | DFD | 15/Dec 98 |
| 6 | Jun 98 | 2 | no | - | DFD | DFD | 3/ |
| 7 | Jul 98 | 5 | no | + | CR | CR | 16/Aug 99 |
| 8 | Jul 98 | 6 | fever | + | PR | CR | 16/Sep 99 |
| 9 | Sep 98 | 5 | no | + | SD | SD | 15/Dec 99 |
| 10 | Sep 98 | 5 | no | + | SD | PD | 14/Jun 99 |
| 11 | Oct 98 | 3 | fever | - | SD | PD | 14/Oct 99 |
| 12 | Nov 98 | 3 | no | + | MR | MR | 13/Feb 99 |
| 13 | Jan 99 | 2 | no | - | DFD | DFD | 3/- |
| 14 | Feb 99 | 3 | no | + | PR | DFD | 6/May 99 |
| 15 | Mar 99 | 3 | no | + | CR | CR | 10/Nov 99 |
| 16 | | 2 | no | + | PR | PR | 9/Sep 99 |
| 17 | | 3 | | - | PD | PD | 8/Jun 99 |
| Ch, chemotherapy with IFN, interlukin-2 and 5-fluoyouracil; S, surgery of metastasis, TN, tumor naphrectomy. Donor HLA class I haplotypes for the first two vaccinations. B, bone; L, lung; Li, liver; LR, local recurrence ; Ly, lymph nodes; Pa, Pancreas; ST, soft tissue, Muc1 expression of the primary tumor, as assessed by Immune Histochemistry. Percent positive cells: 0, neg.; 1-10% + 10-60%, ++; 60-100%, +++. DTH reactions after challenges with irradiated autologous tumor cells; + erythema and induration greater than 6mm in diameter. Clinical response: CR, complete response; PR, partial response; MR 'mixed response'; SD, stable disease; PD, progressive disease; DFD, died from disease. Follow-up. (FU), in months/date of latest CT scan in which the clinical response was documented. | | | | | | | |

## Claims

**1.** A method of treating or preventing a condition in a mammal selected from the group consisting of cancer and an infectious disease, said method comprising administering to a mammal in need of said treatment or prevention a therapeutically effective amount of a hybrid cell formed by the fusion of an allogeneic dendritic cell and a non-dendritic cell which shares at least one class I MHC allele in with said mammal.

**2.** A method of treating a condition in a mammal selected from the group consisting of cancer and an infectious disease, said method comprising administering to a mammal in need of said treatment a therapeutically effective amount of a hybrid cell formed by the fusion of an allogeneic non-dendritic cell and a dendritic cell which shares at least one class I MHC allele with said mammal.

**3.** The method of claim 1 or 2, wherein the dendritic cell is a mature dendritic cell.

**4.** A method of preventing a condition in a mammal selected from the group consisting of cancer and an infectious disease, said method comprising administering to a mammal in need of said treatment a therapeutically effective amount of a hybrid cell formed by the fusion of an allogeneic non-dendritic cell and a mature dendritic cell which has at least one class I MHC allele in common with said mammal.

**5.** The method of claim 1, 2, or 4, wherein the dendritic cell is obtained from human blood monocytes.

**6.** The method of claim 1, wherein the non-dendritic cell is autologous.

**7.** The method of claim 6, wherein the non-dendritic cell is a tumor cell obtained from the mammal.

**8.** The method of claim 6, wherein the non-dendritic cell is a tumor cell line derived from a tumor cell obtained from the mammal in which the hybrid cell is to be administered.

**9.** The method of claim 1, 2, or 4, wherein the non-dendritic cell is a recombinant cell transformed with one or more antigens that display the antigenicity of a tumor-specific antigen.

**10.** The method of claim 1, 2, or 4, wherein the non-dendritic cell is a recombinant cell transformed with one or more antigens that display the antigenicity of an antigen of an infectious agent.

**11.** The method of claim 1, 2, or 4, wherein the mammal is a human.

**12.** The method of claim 1, 2, or 4, wherein the mammal is selected from the group consisting of a cow, a horse, a sheep, a pig, a fowl, a goat, a cat, a dog, a hamster, a mouse and a rat.

**13.** The method of claim 1, 2, or 4, wherein the cancer is selected from the group consisting of renal cell carcinoma, fibrosarcoma, myxosarcoma, liposarcoma, chondrosarcoma, osteogenic sarcoma, chordoma, angiosarcoma, endotheliosarcoma, lymphangiosarcoma, lymphangioendotheliosarcoma, synovioma, mesothelioma, Ewing's tumor, leiomyosarcoma, rhabdomyosarcoma, colon carcinoma, pancreatic cancer, breast cancer, ovarian cancer, prostate cancer, squamous cell carcinoma, basal cell carcinoma, adenocarcinoma, sweat gland carcinoma, sebaceous gland carcinoma, papillary carcinoma, papillary adenocarcinomas, cystadenocarcinoma, medullary carcinoma, bronchogenic carcinoma, hepatoma, bile duct carcinoma, choriocarcinoma, seminoma, embryonal carcinoma, Wilms' tumor, cervical cancer, testicular tumor, lung carcinoma, small cell lung carcinoma, bladder carcinoma, epithelial carcinoma, glioma, astrocytoma, medulloblastoma, craniopharyngioma, ependymoma, pinealoma, hemangioblastoma, acoustic neuroma, oligodendroglioma, meningioma, melanoma, neuroblastoma, retinoblastoma, leukemias, acute lymphocytic leukemia, acute myelocytic leukemia; chronic leukemia, polycythemia vera, lymphoma, multiple myeloma, Waldenström's macroglobulinemia, and heavy chain disease.

**14.** The method of claim 1, 2, or 4, wherein the infectious disease is caused by an intracellular pathogen.

**15.** The method of claim 14, wherein the intracellular pathogen is a virus selected from the group consisting of hepatitis type B virus, parvoviruses, cytomegalovirus, papovaviruses, polyoma viruses, SV40, adenoviruses, herpes viruses, Epstein-Barr virus, poxviruses, vaccinia virus, human immunodeficiency virus type I, human immunodeficiency virus type II, human T-cell lymphotropic virus type I, human T-cell lymphotropic virus type II, influenza virus, measles virus, rabies virus, Sendai virus, picomaviruses, coxsackieviruses, rhinoviruses, reoviruses, togaviruses, rubella virus, Semliki forest virus, arboviruses, and hepatitis type A virus.

**16.** The method of claim 14, wherein the intracellular pathogen is selected from the group consisting of a bacterium, a protozoa, and a parasite.

**17.** A method for preventing a cancer in an individual having a predisposition for the cancer comprising the method of claim 1 or 4, wherein the non-dendritic cell displays the antigenicity of an antigen associated with the cancer.

**18.** The method of claim 17, wherein is the cancer is colon cancer, and the non-dendritic cell is derived from a cell or cell line displaying an antigen associated with colon cancer.

**19.** The method of claim 17, wherein is the cancer is breast cancer, and the non-dendritic cell is derived from a cell or a cell line displaying an antigen associated with breast cancer.

**20.** A method for making a fusion of a mature human dendritic cell and a non-dendritic human cell comprising subjecting a population of mature dendritic cells and a population of non-dendritic cells allogeneic to the dendritic cells to conditions that promote cell fusion.

**21.** The method of claim 20 further comprising the step of inactivating the population of hybrid cells.

**22.** The method of claim 20 wherein the cell fusion is accomplished by electrofusion.

**23.** The method of claim 20 wherein the inactivating the population of hybrid cells is accomplished by γ-irradiating the cells.

**24.** A kit comprising, in one or more containers, a sample containing a population of mature dendritic cells and instructions for its use in treating or preventing cancer or an infectious disease.

**25.** A kit comprising, in one or more containers, a sample containing a population of mature dendritic cells and instructions for its use in making a fusion with a non-dendritic cell.

**26.** The kit of claim 24 or 25 further comprising a cuvette suitable for electrofusion.

**27.** The kit of claim 24 or 25 wherein the dendritic cells are cryopreserved.

**28.** A hybrid cell comprising a mature dendritic cell fused to a non-dendritic cell, which non-dendritic cell is freshly isolated or obtained from a primary cell culture, wherein the dendritic cell and non-dendritic cell are allogeneic to each other.

**22.** The hybrid cell of claim 28 wherein the mature dendritic cell and the non-dendritic cell are human.

**23.** The hybrid cell of claim 28 wherein the non-dendritic cell is a tumor cell.
